(19) **Europäisches Patentamt / European Patent Office / Office européen des brevets**

(11) **EP 2 786 397 B1**

(12) **FASCICULE DE BREVET EUROPEEN**

(45) Date de publication et mention
de la délivrance du brevet:
**02.10.2019 Bulletin 2019/40**

(21) Numéro de dépôt: **12798884.8**

(22) Date de dépôt: **30.11.2012**

(51) Int Cl.:
*H01J 49/00* $^{(2006.01)}$ *C12Q 1/04* $^{(2006.01)}$

(86) Numéro de dépôt international:
**PCT/IB2012/056860**

(87) Numéro de publication internationale:
**WO 2013/080170 (06.06.2013 Gazette 2013/23)**

(54) **PROCEDE D'IDENTIFICATION DE MICROORGANISMES PAR SPECTROMETRIE DE MASSE**

VERFAHREN ZUR IDENTIFIZIERUNG VON MIKROORGANISMEN DURCH
MASSENSPEKTROMETRIE

METHOD FOR IDENTIFYING MICROORGANISMS BY MASS SPECTROMETRY

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **02.12.2011 EP 11306610
02.12.2011 US 201161566025 P**

(43) Date de publication de la demande:
**08.10.2014 Bulletin 2014/41**

(73) Titulaire: **Biomérieux Inc.
Durham, North Carolina 27712 (US)**

(72) Inventeurs:
• **STRUBEL, Grégory
F-04100 Manosque (FR)**
• **ARSAC, Maud
F-42400 Saint Chamond (FR)**
• **DESSEREE, Denis
F-01120 Montluel (FR)**
• **COTTE-PATTAT, Pierre-Jean
F-01150 Lagnieu (FR)**

(74) Mandataire: **Le Mauff, Frédéric Francis Joseph
bioMérieux
376, chemin de l'Orme
69280 Marcy-l'Étoile (FR)**

(56) Documents cités:
**US-A- 4 008 388      US-A1- 2004 195 500
US-A1- 2008 029 697**

• **SCOTT C RUSSELL: "MICROORGANISM
CHARACTERIZATION BY SINGLE PARTICLE
MASS SPECTROMETRY", MASS
SPECTROMETRY REVIEWS, JOHN WILEY AND
SONS, NEW YORK, NY, US, vol. 28, 1 janvier 2009
(2009-01-01), pages 376-387, XP007910439, ISSN:
0277-7037, DOI: 10.1002/MAS**
• **JUSTIN M. HETTICK ET AL: "Proteomic Profiling
of Intact Mycobacteria by Matrix-Assisted Laser
Desorption/Ionization Time-of-Flight Mass
Spectrometry", ANALYTICAL CHEMISTRY, vol.
76, no. 19, 1 octobre 2004 (2004-10-01), pages
5769-5776, XP055031885, ISSN: 0003-2700, DOI:
10.1021/ac049410m**
• **KEYS C J ET AL: "Compilation of a MALDI-TOF
mass spectral database for the rapid screening
and characterisation of bacteria implicated in
human infectious diseases", INFECTION,
GENETICS AND EVOLUTION, ELSEVIER,
AMSTERDAM, NL, vol. 4, no. 3, 1 September 2004
(2004-09-01), pages 221-242, XP027554585, ISSN:
1567-1348 [retrieved on 2004-08-21]**

EP 2 786 397 B1

Printed by Jouve, 75001 PARIS (FR)

**Description**

**DOMAINE DE L'INVENTION**

L'invention a trait au domaine de l'identification de microorganisme, et notamment des bactéries, au moyens de la spectrométrie de masse.

**ETAT DE LA TECHNIQUE**

Il est connu d'utiliser la spectrométrie de masse pour identifier des micro-organismes, et plus particulièrement des bactéries. Un échantillon du micro-organisme est préparé puis un spectre de masse de l'échantillon est acquis et prétraité : débruitage du spectre (élimination du bruit), filtrage du bruit de fond (attribuable à la surcharge du détecteur). Les pics importants du spectre prétraité sont alors détectés et la liste de pics ainsi obtenue est « analysée » et « comparée » à des données d'une base de connaissances construite à partir de listes de pics typiques d'un microorganisme ou d'un groupe de micro-organismes (souche, genre, famille, etc...) identifié.
Si le principe parait simple a priori, sa mise en oeuvre est toutefois délicate. En effet, tout d'abord, la quantité d'information contenue dans un spectre de masse, notamment le nombre de pics, est très importante, ce qui nécessite des outils de calcul très puissants pour créer une base de connaissance robuste, ainsi que pour la mise en oeuvre d'algorithmes de classification, de comparaison et de décision.
Ensuite, il existe une incertitude de mesure importante, notamment en ce qui concerne la localisation des pics dans le spectre. On observe en effet que d'une mesure à l'autre sur un même spectromètre, ainsi que d'un spectromètre à l'autre, un pic représentant une molécule donnée n'a pas une position fixe dans les spectres mesurés, ou à tout le moins le pic n'est pas contenu dans une plage. Ainsi, un pic d'un spectre acquis, et correspondant à une molécule de protéine donnée, peut ne pas être identifié comme correspondant à ladite molécule de protéine par l'algorithme de classification. Enfin, cette incertitude n'est pas constante sur la gamme de rapports masse-sur-charge et augmente à mesure que ce rapport augmente.
Le document US2004/0195500 décrit un procédé d'analyse de spectre de masse de matériel biologique comprenant une étape de binning logarithmique de l'intervalle m/z.

**EXPOSE DE L'INVENTION**

Le but de l'invention est de proposer un procédé permettant l'identification robuste des micro-organismes par spectrométrie de masses grâce à une réduction de la masse d'informations à analyser et une réduction de l'impact de l'absence de précision dans la localisation des pics du spectre de masse.
A cet effet, l'invention a pour objet un procédé d'identification d'un microorganisme par spectrométrie de masse, comprenant :

- l'acquisition d'au moins un spectre de masse dudit microorganisme ;
- pour chaque spectre de masse acquis :

  ◦ la détection de pics du spectre dans une gamme prédéterminée de masses ;
  ◦ la génération d'une liste de pics répertoriant au plus un pic dans chaque intervalle d'une subdivision prédéterminée de la gamme des rapports masse-sur-charge, la largeur des intervalles de la subdivision augmentant avec le rapport masse-sur-charge selon les relations :

$$L(b) = \exp\left(\frac{b-\beta}{\alpha}\right) \times \left(\exp\left(\frac{1}{\alpha}\right) - 1\right)$$

$$\alpha = \frac{b_{\min} - (b_{\max} + 1)}{\ln m_{\min} - \ln m_{\max}}$$

$$\beta = \frac{(b_{\max} + 1) \times \ln m_{\min} - b_{\min} \times \ln m_{\max}}{\ln m_{\min} - \ln m_{\max}}$$

où les intervalles de la subdivision sont référencés par des entiers supérieurs à 1 depuis l'entier $b_{min}$, pour les rapports masses-sur-charges les plus faibles de la gamme, à l'entier $b_{max}$, pour les rapports masses-sur-charges les plus importants de la gamme, $L(b)$ est la largeur de l'intervalle référencé par l'entier $b$, $m_{min}$ est une borne inférieure de la gamme des rapports masse-sur-charge, et $m_{max}$ est une borne supérieure de la gamme des rapports masse-sur-charge; et

- l'analyse de la ou des listes de pics obtenues en fonction de la base de connaissances de microorganismes et/ou à de types de microorganismes préalablement identifiés.

En d'autres termes, l'espace continu des rapports masse-sur-charge, ou Thompson, est quantifié de manière logarithmique, et il n'est retenu qu'un seul pic dans chaque intervalle de quantification si plusieurs pics sont présents dans cet intervalle. Ceci permet une réduction sensible de la quantité de données à traiter. En outre, la position précise d'un pic est remplacée par la référence de l'intervalle auquel le pic appartient. Ceci réduit l'incertitude de mesure concernant la position de pics puisqu'il n'est plus besoin de comparer une position précise avec la base de connaissance. Il est plutôt comparé l'appartenance du pic mesuré à un intervalle. Enfin, la progression logarithmique de la largeur des intervalles permet de s'adapter au fait que l'instrument présente une précision relative constante :

$$p = \frac{\Delta\mu}{m} = \text{constante}$$

Selon un mode de réalisation, la gamme prédéterminée de Thompsons est comprise entre 3000 Thompsons et 17000 Thompsons. Les inventeurs ont en effet constaté que cette gamme était suffisante pour l'identification de la plupart des bactéries et des levures/moisissures. On observe notamment que les pics localisés en dessous de 3000 Thompsons sont communs à beaucoup de microorganisme et ne sont donc pas discriminants.

Selon l'invention, le nombre d'intervalles est compris entre 900 et 1500, notamment entre 1200 et 1400. Les inventeurs ont constaté que ces intervalles constituent le compromis optimal entre la perte d'informations induite par la quantification de l'espace des Thompsons et la précision gagnée par le remplacement de la position précise des pics par les intervalles.

Selon un mode de réalisation, le pic gardé dans un intervalle de la subdivision est le pic avec l'intensité la plus forte. D'autres choix sont cependant possibles. Par exemple, il est possible de choisir la valeur moyenne ou la valeur médiane des intensités des pics présents dans l'intervalle.

Selon un mode de réalisation, la spectrométrie de masse est une spectrométrie MALDI-TOF.

L'invention a également pour objet un dispositif d'identification d'un microorganisme par spectrométrie de masse, comprenant :

- un spectromètre de masse apte à produire des spectres de masse de microorganismes à identifier ;
- une unité de calcul apte à identifier les microorganismes associés aux spectres de masse produits par le spectromètre en mettant en oeuvre un procédé conforme à l'un des modes de réalisation précédents.

**BREVE DESCRIPTION DES FIGURES**

L'invention sera mieux comprise à la lecture de la description qui va suivre, donnée uniquement à titre d'exemple, et faire en relation avec les dessins annexés, dans lequel :

- la figure 1 est un organigramme du procédé selon l'invention ; et
- la figure 2 est un tracé du nombre de pics éliminés dans un spectre de masse en fonction du nombre d'intervalles de la quantification selon l'invention.

**DESCRIPTION DETAILLEE DE L'INVENTION**

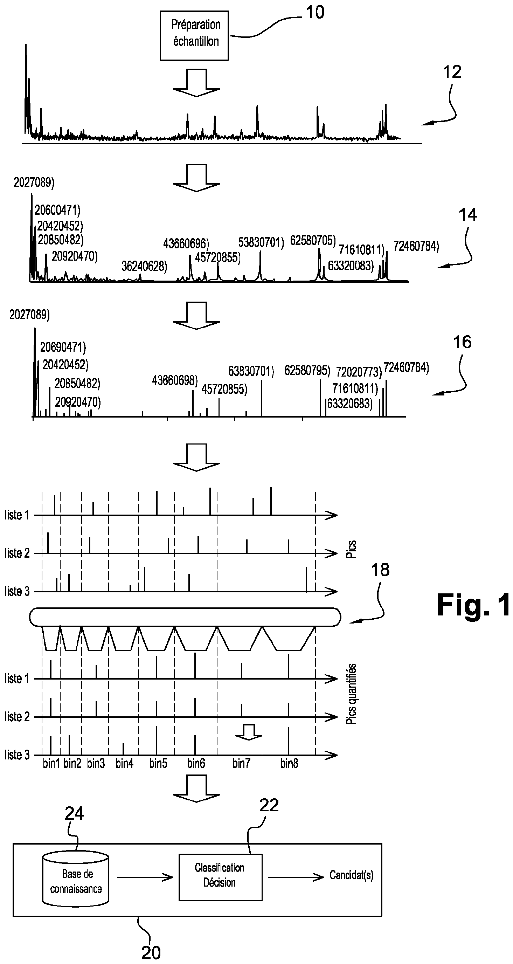

Il va à présent être décrit en référence avec la figure 1 un procédé selon l'invention d'identification de bactéries au moyen d'une spectrométrie de masse de type MALDI-TOF (acronyme de « *Matrix-assisted laser desorption/ionizcition time of flight* »).

Le procédé débute par la préparation, en 10, d'un échantillon d'une bactérie à identifier, suivi de l'acquisition, en 12, d'un ou plusieurs spectres de masse de l'échantillon préparé au moyen d'un spectromètre de masse de type MALDI-TOF. La spectrométrie de masse MALDI-TOF est bien connue en soi et ne sera donc pas décrite plus en détail par la suite. On pourra, par exemple, se référer au document de Jackson O. Lay, « " Maldi-tof spectrometry of bacteria », Mass Spectrometry Reviews, 2001, 20, 172-194.

Le procédé se poursuit, en 14, par le prétraitement des spectres acquis, afin notamment de débruiter et lisser les spectres. Plus particulièrement, la ligne de base des spectres, qui représente le bruit de fond du spectromètre est retirée.

Une identification des pics présents dans les spectres acquis est alors réalisée en 16, par exemple au moyen d'un algorithme détection de pics par exemple basé sur la détection de maximums locaux. Une liste des pics pour chaque spectre, comportant la localisation et l'intensité des pics du spectre, est ainsi produite.

De manière avantageuse, les pics sont identifiés dans la gamme de Thompsons $[m_{min} ; m_{max}]$ restreinte, de préférence la gamme $[m_{min} ; m_{max}] = [3000; 17000]$ Thompsons. En effet, il a été observé que les informations suffisantes à l'identification des micro-organismes sont regroupées dans cette gamme, et qu'il n'est donc pas besoin de tenir compte d'une gamme plus large.

Le procédé se poursuit, en 18, par une étape de quantification, ou « binning ». Pour ce faire :

- la gamme $[m_{min} ; m_{max}]$ est subdivisée en intervalles dont la largeur croit de manière logarithmique avec les Thompsons selon la relation :

$$L(b) = \exp\left(\frac{b-\beta}{\alpha}\right) \times \left(\exp\left(\frac{1}{\alpha}\right) - 1\right) \qquad (1)$$

$$\alpha = \frac{b_{min} - (b_{max} + 1)}{\ln m_{min} - \ln m_{max}} \qquad (2)$$

$$\beta = \frac{(b_{max} + 1) \times \ln m_{min} - b_{min} \times \ln m_{max}}{\ln m_{min} - \ln m_{max}} \qquad (3)$$

où les intervalles de la subdivision sont référencés par des entiers supérieurs à 1 depuis l'entier $b_{min}$, par exemple égal à 1, à l'entier $b_{max}$, et $L(b)$ est la largeur de l'intervalle référencé par l'entier $b$. L'entier $b_{min}$ correspond à l'intervalle des rapports masses-sur-charges les plus faibles de la gamme $[m_{min} ; m_{max}]$, et l'entier $b_{max}$ correspond à l'intervalle des rapports masses-sur-charges les plus importants de la gamme $[m_{min} ; m_{max}]$. L'axe des Thompsons est donc quantifié selon les relations :

$$b(m) = \lfloor \alpha \ln m + \beta \rfloor$$

où $\lfloor \rfloor$ est le symbole de l'arrondi à l'entier inférieur ;
- pour chaque intervalle comprenant plusieurs pics, un seul pic est conservé, avantageusement le pic présentant la plus forte intensité. Un vecteur est ainsi produit pour chaque spectre mesuré. Chaque composante du vecteur correspond à un intervalle de la quantification et a pour valeur l'intensité du pic conservé pour cet intervalle, la valeur « 0 » signifiant qu'aucun pic n'a été détecté dans cet intervalle.

Par exemple, à l'étape 18 de la figure sur la figure 1, il est illustré trois listes de pics identifiés, à savoir « liste 1 », « liste 2 » et « liste 3 », correspondant chacune à un spectre de masse mesuré. L'espace des Thompsons est subdivisé en 8 intervalles, de « bin1 » à « bin8 », de largeur croissante de façon logarithmique, et seul le pic de plus forte intensité est conservé dans chaque intervalle. Ainsi, pour l'intervalle « bin6 » de la première liste « liste 1 », un pic est éliminé. Pour les listes « liste 1 », « liste 2 » et « liste 3 », il est par exemple obtenu la matrice suivante, chaque ligne correspondant à une liste :

$$\begin{pmatrix} 980 & 0 & 98 & 0 & 1300 & 1556 & 400 & 2000 \\ 505 & 700 & 200 & 0 & 500 & 200 & 345 & 256 \\ 700 & 0 & 0 & 100 & 2340 & 1786 & 0 & 2507 \end{pmatrix}$$

On montre ainsi qu'à l'aide d'une quantification telle que décrite précédemment, il est tenu compte de l'accroissement de l'incertitude sur la position des pics à mesure que les masses augmentent. Notamment, la subdivision selon l'invention de l'axe des Thompsons permet de tenir compte d'une incertitude du type :

$$p = \frac{\Delta\mu}{m} \qquad (4)$$

où $p$ est la précision sur la localisation d'un pic, $\Delta\mu$ est l'incertitude de mesure sur la position des pics du spectromètre, et $m$ est la position réelle du pic. La quantification est donc une quantification adaptative qui tient compte de l'erreur de mesure du spectromètre de masse.

Le remplacement de la localisation mesurée d'un pic par la référence à l'intervalle auquel il appartient, est équivalent à aligner la position du pic sur le milieu de l'intervalle. On vérifie que la subdivision logarithmique selon l'invention permet de réduire l'incertitude selon la relation (4). En effet, on a :

$$\frac{L(b)}{m_{bar}(b)} = \frac{2\left(\exp\left\{\frac{1}{\alpha}\right\} - 1\right)}{\left(\exp\left\{\frac{1}{\alpha}\right\} + 1\right)} = \mathrm{cte}$$

où $m_{bar}(b)$ est le milieu de l'intervalle référencé de référence $b$.

L'intensité d'un pic est très variable d'un spectre à l'autre et/ou d'un spectromètre à un autre. Du fait de cette variabilité, il est très difficile de prendre en compte les valeurs brutes d'intensité. De manière avantageuse, mais optionnelle, le procédé se poursuit par une étape de discrétisation des intensités. Cette étape peut par exemple consister en une simple « binarisation » (présence/absence).

Ainsi, chaque ligne de la matrice est « binarisée » puis normalisée, la matrice répertoriant ainsi pour chaque spectre acquis la présence ou l'absence de pic dans les intervalles. Par exemple, la matrice précédente est binarisée en la matrice :

$$\begin{pmatrix} 1 & 0 & 1 & 0 & 1 & 1 & 1 & 1 \\ 1 & 1 & 1 & 0 & 1 & 1 & 1 & 1 \\ 1 & 0 & 0 & 1 & 1 & 1 & 0 & 1 \end{pmatrix}$$

Les inventeurs ont en outre noté que l'information pertinente pour l'identification d'une bactérie est contenue essentiellement dans l'absence et/ou la présence de pics, et que l'information d'intensité est moins pertinente, notamment en raison de sa forte variabilité. Ainsi par exemple, il est possible d'identifier des bactéries sur la base de ce type de listes à l'aide d'outils de classification usuels tels que la régression logistique, l'analyse discriminante, les arbres de classification, méthodes LASSO, des algorithmes de type SVM (acronyme de l'expression anglo-saxonne « *support vector machine* »). La matrice ainsi binarisée peut être utilisée dans tous les outils de classification connus.

Le procédé se poursuit ensuite, en 20, par l'analyse de la matrice obtenue à l'étape précédente. Plus particulièrement, un algorithme de classification et de décision 22 est mis en oeuvre en fonction d'une base de connaissances 24 construite en fonction de listes de pics de microorganismes et/ou types de microorganismes préalablement identifiés. Un ou plusieurs candidats, ou un type de microorganismes (famille, germe, espèce, sous-espèces) sont ainsi identifiés pour l'échantillon analysé.

Le procédé selon l'invention permet ainsi de ramener une liste de pics de taille variable et à valeurs continues sur 2 axes (m/z, intensités) à un vecteur de taille fixe et raisonnable.

La base de connaissances 24 est construite à partir de listes de pic produites comme décrit ci-dessus, et associées à des microorganismes et/ou types de microorganismes préalablement identifiés. On comprendra que l'invention s'applique à tout type d'algorithmes de classification et de bases de connaissances. La quantification selon l'invention permet notamment de réduire la quantité de données, ainsi que d'éliminer les problèmes de précision dans la localisation des pics, et permet donc de construire une base de connaissances plus robuste, et ce de manière plus simple. La mise en oeuvre est beaucoup plus simple que le calcul d'une distance tolérante (par exemple) et permet une construction quasi-automatisée de la base de connaissance

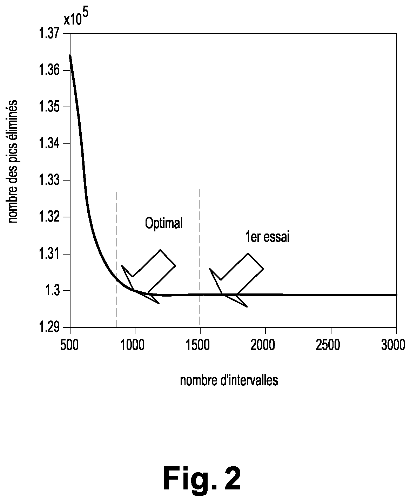

Le nombre d'intervalles est avantageusement choisi entre 900 et 1500, et de préférence entre 1200 et 1400 pour l'identification de micro-organismes. Les inventeurs ont constaté que ces intervalles constituent le compromis optimal entre la perte d'informations induite par la quantification de l'espace des Thompsons et la précision gagnée par le remplacement de la position précise des pics par les intervalles. Les inventeurs ont procédé à des essais et ont modélisé, comme illustré à la figure 2, le nombre de pics éliminés par la quantification en fonction du nombre d'intervalles. On remarque notamment qu'au-delà d'un certain nombre d'intervalles, la réduction de la quantité de donnée est minime, et qu'en deçà d'un certain nombre, le nombre de pics éliminés croit de manière exponentielle.

Il a été procédé à des essais comparatifs entre la quantification logarithmique de l'invention et une quantification constante, c'est-à-dire une quantification pour laquelle tous les intervalles sont de largeur identique, et ce pour un même spectromètre de masse et un algorithme de classification et de décision et une construction de base de connaissance identiques. Ces essais sont décrits dans le tableau ci-dessous. L'erreur correspond à l'erreur d'identification des microorganismes.

| Quantification | Nombre d'intervalles | Erreur (en %) | Espace mémoire occupé (en Mo) |
| --- | --- | --- | --- |
| logarithmique | 300 | 10,33 | 120 |
| logarithmique | 600 | 6,25 | 240 |
| logarithmique | 800 | 5,3 | 320 |
| logarithmique | 1000 | 5,4 | 400 |
| logarithmique | 1300 | 5,0 | 520 |
| logarithmique | 1700 | 6,9 | 680 |
| logarithmique | 2300 | 8,52 | 920 |
| logarithmique | 4700 | 12,2 | 1880 |
| constante | 300 | 12,4 | 120 |
| constante | 600 | 8,75 | 240 |
| constante | 800 | 7,2 | 320 |
| constante | 1000 | 6,6 | 400 |
| constante | 1300 | 6,2 | 520 |
| constante | 1700 | 5,9 | 680 |
| constante | 2300 | 7,22 | 920 |
| constante | 4700 | 11,0 | 1880 |

En choisissant 1000 intervalles de largeur constante, la largeur des intervalles est égale à la résolution du spectromètre de masse utilisé pour les essais pour un rapport masse-sur-charge égal à 17 000 Thompsons. En choisissant, 4700 intervalles de largeur constante, la largeur des intervalles est égale à la résolution du spectromètre pour un rapport masse-sur-charge égal à 3000 Thompsons.

En choisissant 1700 intervalles logarithmiques selon les relations (1) à (3), avec $b_{min}$ = 1, la largeur de chaque intervalle est égale à la précision du spectromètre pour un rapport masse-sur-charge égal au milieu de l'intervalle. Toutefois, il est observé, qu'en moyenne, un nombre de 1300 intervalles permet d'obtenir à la fois le taux d'erreur d'identification le plus bas et l'espace mémoire occupé le plus bas. Notamment, par rapport au nombre de 1700 intervalles, qui semble au premier abord le plus adapté, un gain de 2 points d'erreur (-28% d'erreur) est réalisé, tout en diminuant l'espace mémoire occupé, comme l'indique le tableau ci-dessus. Le nombre de 1300 est donc privilégié pour la mise en oeuvre de l'invention.

On remarque également que la quantification selon l'invention permet d'avoir un taux d'erreur maximale inférieur d'au moins 1 point (-15% d'erreur) à celui d'une quantification constante, ainsi qu'une plus faible empreinte mémoire (-25%). Pour des nombres d'intervalles faibles, la quantification selon l'invention donne ainsi de meilleurs résultats qu'une quantification constante. Ceci permet ainsi de garder un nombre faible d'intervalles, même en augmentant la résolution du spectromètre de masse ou la plage de Thompsons retenue [$m_{min}$ ; $m_{max}$]. Ainsi, on remarque que pour un même taux d'erreur, par exemple environ 6%, la quantification selon l'invention nécessite uniquement 700 intervalles alors que la quantification constante en nécessite 1700.

**Revendications**

1. Procédé d'identification d'un microorganisme par spectrométrie de masse, comprenant :

- l'acquisition d'au moins un spectre de masse dudit microorganisme ;
- pour chaque spectre de masse acquis :

◦ la détection de pics du spectre dans une gamme prédéterminée de masses ;
◦ la génération d'une liste de pics répertoriant au plus un pic dans chaque intervalle d'une subdivision prédéterminée de la gamme des rapports masse-sur-charge, la largeur des intervalles de la subdivision augmentant avec le rapport masse-sur-charge selon les relations :

$$L(b) = \exp\left(\frac{b - \beta}{\alpha}\right) \times \left(\exp\left(\frac{1}{\alpha}\right) - 1\right)$$

$$\alpha = \frac{b_{\min} - (b_{\max} + 1)}{\ln m_{\min} - \ln m_{\max}}$$

$$\beta = \frac{(b_{\max} + 1) \times \ln m_{\min} - b_{\min} \times \ln m_{\max}}{\ln m_{\min} - \ln m_{\max}}$$

où les intervalles de la subdivision sont référencés par des entiers supérieurs à 1 depuis l'entier $b_{\min}$, pour les rapports masses-sur-charges les plus faibles de la gamme, à l'entier $b_{\max}$, pour les rapports masses-sur-charges les plus importants de la gamme, $L(b)$ est la largeur de l'intervalle référencé par l'entier $b$, $m_{\min}$ est une borne inférieure de la gamme des rapports masse-sur-charge, et $m_{\max}$ est une borne supérieure de la gamme des rapports masse-sur-charge; et

▪ l'analyse de la ou des listes de pics obtenues en fonction d'une base de connaissances de microorganismes et/ou à de types de microorganismes préalablement identifiés,

procédé dans lequel le nombre d'intervalles est compris entre 900 et 1500.

**2.** Procédé selon la revendication 1, dans lequel la gamme prédéterminée des rapports masse-sur-charge est comprise entre 3000 Thompsons et 17000 Thompsons.

**3.** Procédé selon la revendication 1 ou 2, dans lequel le nombre d'intervalles est compris entre 1200 et 1400.

**4.** Procédé selon l'une quelconque des revendications précédentes, dans lequel le pic gardé dans un intervalle de la subdivision est le pic maximum.

**5.** Procédé selon l'une quelconque des revendications précédentes, dans lequel la spectrométrie de masse est une spectrométrie MALDI-TOF.

**6.** Dispositif d'identification d'un microorganisme par spectrométrie de masse, comprenant :

▪ un spectromètre de masse apte à produire des spectres de masse de microorganismes à identifier ;
▪ une unité de calcul apte à identifier les microorganismes associés aux spectres de masse produits par le spectromètre en mettant en oeuvre un procédé conforme à l'une quelconque des revendications précédentes.

**Patentansprüche**

**1.** Verfahren zur Identifizierung von Mikroorganismen durch Massenspektrometrie, welches umfasst:

▪ die Erfassung wenigstens eines Massenspektrums des Mikroorganismus;
▪ für jedes erfasste Massenspektrum:

◦ die Detektion von Peaks des Spektrums in einem vorbestimmten Massenbereich;
◦ die Erzeugung einer Liste von Peaks, in der höchstens ein Peak in jedem Intervall einer vorbestimmten Aufteilung des Bereichs der Masse-zu-Ladung-Verhältnisse verzeichnet ist, wobei die Breite der Intervalle der Aufteilung mit dem Masse-zu-Ladung-Verhältnis gemäß den Beziehungen zunimmt:

$$L(b) = \exp\left(\frac{b - \beta}{\alpha}\right) \times \left(\exp\left(\frac{1}{\alpha}\right) - 1\right)$$

$$\alpha = \frac{b_{\min} - (b_{\max} + 1)}{\ln m_{\min} - \ln m_{\max}}$$

$$\beta = \frac{(b_{\max} + 1) \times \ln m_{\min} - b_{\min} \times \ln m_{\max}}{\ln m_{\min} - \ln m_{\max}}$$

wobei die Intervalle der Aufteilung mit ganzen Zahlen größer als 1 von der ganzen Zahl $b_{\min}$ für die kleinsten Masse-zu-Ladung-Verhältnisse des Bereichs bis zur ganzen Zahl $b_{\max}$ für die größten Masse-zu-Ladung-Verhältnisse des Bereichs bezeichnet werden, $L(b)$ die Breite des mit der ganzen Zahl $b$ bezeichneten Intervalls ist, $m_{\min}$ eine untere Grenze des Bereichs der Masse-zu-Ladung-Verhältnisse ist und $m_{\max}$ eine obere Grenze des Bereichs der Masse-zu-Ladung-Verhältnisse ist; und

■ die Analyse der erhaltenen Liste oder Listen von Peaks in Abhängigkeit von einer Wissensdatenbank von Mikroorganismen und/oder Typen von Mikroorganismen, die zuvor identifiziert wurden, wobei die Anzahl von Intervallen bei dem Verfahren zwischen 900 und 1500 liegt.

2. Verfahren nach Anspruch 1, wobei der vorbestimmte Bereich der Masse-zu-Ladung-Verhältnisse zwischen 3000 Thomson und 17000 Thomson liegt.

3. Verfahren nach Anspruch 1 oder 2, wobei die Anzahl von Intervallen 1200 und 1400 liegt.

4. Verfahren nach einem der vorhergehenden Ansprüche, wobei der in einem Intervall der Aufteilung beibehaltene Peak der maximale Peak ist.

5. Verfahren nach einem der vorhergehenden Ansprüche, wobei die Massenspektrometrie eine MALDI-TOF-Spektrometrie ist.

6. Vorrichtung zur Identifizierung von Mikroorganismen durch Massenspektrometrie, welche umfasst:

■ ein Massenspektrometer, das geeignet ist, Massenspektren von zu identifizierenden Mikroorganismen zu erzeugen;
■ eine Berechnungseinheit, die geeignet ist, die Mikroorganismen zu identifizieren, die den von dem Massenspektrometer erzeugten Massenspektren zugeordnet sind, indem sie ein Verfahren gemäß einem der vorhergehenden Ansprüche durchführt.

## Claims

1. A method of identifying a microorganism by mass spectrometry, comprising:

■ acquiring at least one mass spectrum of said microorganism;
■ for each acquired mass spectrum:

◦ detecting peaks of the spectrum in a predetermined mass range;
◦ generating a list of peaks identifying at most one peak in each interval of a predetermined subdivision of the range of mass-to-charge ratios, the width of the intervals of the subdivision increasing along with the mass-to-charge ratio according to relations:

$$L(b) = \exp\left(\frac{b-\beta}{\alpha}\right) \times \left(\exp\left(\frac{1}{\alpha}\right) - 1\right)$$

$$\alpha = \frac{b_{min} - (b_{max} + 1)}{\ln m_{min} - \ln m_{max}}$$

$$\beta = \frac{(b_{max} + 1) \times \ln m_{min} - b_{min} \times \ln m_{max}}{\ln m_{min} - \ln m_{max}}$$

where the subdivision intervals are referenced with integers greater than 1, from integer $b_{min}$, for the lowest mass-to-charge ratios in the range, to integer $b_{max}$, for the higher mass-to-charge ratios in the range, $L(b)$ is the width of the interval referenced with integer- $b$, $m_{min}$ is a lower bound of the range of mass-to-charge ratios, and $m_{max}$ is an upper bound of the range of mass-to-charge ratios; and

- analyzing the list(s) of peaks obtained according to a knowledge base of previously-identified microorganisms and/or types of microorganisms,

method wherein there are from 900 to 1,500 intervals.

2. The method of claim 1, wherein the predetermined range of mass-to-charge ratios is in the range from 3,000 Thomsons to 17,000 Thomsons.

3. The method of claim 1 or 2, wherein there are from 1,200 to 1,400 intervals.

4. The method of any of the foregoing claims, wherein the peak kept in an interval of the subdivision is the maximum peak.

5. The method of any of the foregoing claims, wherein the mass spectrometry is a MALDI-TOF spectrometry.

6. A device for identifying a microorganism by mass spectrometry, comprising:

- a mass spectrometer capable of generating mass spectrums of microorganisms to be identified;
- a calculation unit capable of identifying the microorganisms associated with the mass spectrums generated by the spectrometer by implementing the method of any of the foregoing claims.

**Fig. 1**

**Fig. 2**

## RÉFÉRENCES CITÉES DANS LA DESCRIPTION

**Documents brevets cités dans la description**

- US 20040195500 A

**Littérature non-brevet citée dans la description**

- **JACKSON O. LAY.** Maldi-tof spectrometry of bacteria. *Mass Spectrometry Reviews,* 2001, vol. 20, 172-194